(19) Europäisches Patentamt

European Patent Office

Office européen des brevets

(11) Veröffentlichungsnummer: **0 186 025 B1**

(12) **EUROPÄISCHE PATENTSCHRIFT**

(45) Veröffentlichungstag der Patentschrift: **12.06.91**

(51) Int. Cl.⁵: **A61K 7/48, A61K 7/06**

(21) Anmeldenummer: 85115631.5

(22) Anmeldetag: 09.12.85

Die Akte enthält technische Angaben, die nach dem Eingang der Anmeldung eingereicht wurden und die nicht in dieser Patentschrift enthalten sind.

(54) **Kosmetische Zubereitungen mit Mandel-Proteinhydrolysat.**

(30) Priorität: 17.12.84 DE 3445919

(43) Veröffentlichungstag der Anmeldung:
02.07.86 Patentblatt 86/27

(45) Bekanntmachung des Hinweises auf die
Patenterteilung:
12.06.91 Patentblatt 91/24

(84) Benannte Vertragsstaaten:
AT BE CH DE FR GB IT LI NL SE

(56) Entgegenhaltungen:

CHEMISCHES ZENTRALBLATT, Nr. 20, 1964,
Seite 299, Zusammenfassung Nr. 2763; O.
GIAMPAOLI geb. LUCCHINI: "Kosmetische
creme"

H. JANISTYN: "Handbuch der Kosmetika und
Riechstoffe", Band 1, 1978, Seite 595, 3. verbesserte und erweiterte Auflage, Dr. Alfred
Hüthig Verlag Heidelberg, DE

Brockhaus' Konversationslexikon Band 11,
14. Auflage, S. 535

(73) Patentinhaber: **Henkel Kommanditgesellschaft
auf Aktien**
**Postfach 1100 Henkelstrasse 67**
**W-4000 Düsseldorf-Holthausen(DE)**

(72) Erfinder: **Ansmann, Achim, Dr**
**Fichtestrasse 19**
**W-4010 Hilden(DE)**

## Beschreibung

Gegenstand der Erfindung sind kosmetische Zubereitungen zur Pflege von Haut und Haaren mit einem Gehalt an Mandel-Proteinhydrolysat.

Es ist bekannt, daß wasserlösliche Proteine und Proteinabbauprodukte, die z.B. durch Hydrolyse oder Aminolyse aus wasserunlöslichen Proteinen gewonnen werden können, einen kosmetisch günstigen Einfluß auf die äußeren Schichten der Haut und das Keratin des Haars haben. Es ist daher auch bekannt, wasserlösliche Proteine oder durch Hydrolyse aus Proteinen gewonnene Oligo- und Polypeptide kosmetischen Zubereitungen zur Pflege der Haut und der Haare zuzusetzen (vgl. z.B. Seifen, Öle, Fette, Wachse 108, Nr. 7 (1982), 177 - 184). Wasserlösliche Proteinhydrolysate besitzen eine gewisse Substantivität zur Haut und zum Haar und üben auf diese Weise eine kolloidale Schutzwirkung, insbesondere gegenüber der entfettenden und quellenden Wirkung der Tenside auf das Haut- und Haarkeratin aus.

Bisher wurden überwiegend Faserproteine, z.B. Keratin, Kollagen und Elastin zur technischen Herstellung wasserlöslicher Hydrolysate für kosmetische Anwendungen eingesetzt. Von den pflanzlichen Proteinen wurde bisher nur Sojaprotein für die Herstellung kosmetisch anwendbarer Hydrolysate vorgeschlagen. Die mit den bekannten Proteinhydrolysaten erzielbaren kosmetischen Effekte sind jedoch so schwach, daß auch weiterhin das Bedürfnis bestand, Proteine mit verbesserter kosmetischer Wirkung aufzufinden.

Es wurde nun gefunden, daß die hautschützenden und haarkosmetischen Effekte in besonders hohem Maße und darüber hinaus deutliche hautfeuchthaltende Wirkungen durch kosmetische Zubereitungen zur Reinigung und Pflege von Haut und Haaren erzielt werden, die ein Mandel-Proteinhydrolysat enthalten.

Mandeln, die Früchte des Mandelbaumes (prumus amygdalus, amygdalus communis) werden nach Entfernung der harten Schale zur Gewinnung von Mandelöl gepreßt. Der Preßkuchen wird gemahlen, gesiebt und unter anderem auch als Mandelkleie (oder Mandelmehl) in der Kosmetik als Hautpflegemittel verwendet. Mandelkleie ist jedoch nicht wasserlöslich und daher nicht universell in wäßrigen Zubereitungen einsetzbar. Ihre kosmetischen Wirkungen sind darüber hinaus nur schwach ausgeprägt, so daß es als überraschend anzusehen ist, daß die Hydrolysate der Mandelproteine wesentlich stärkere kosmetische Wirkungen entfalten. Durch die vorliegende Erfindung wird also ein neuer Weg aufgezeigt, die offenbar in der Mandelkleie enthaltenen kosmetisch wirksamen Inhaltsstoffe für eine universelle Anwendung in kosmetischen Zubereitungen zur Pflege von Haut und Haaren zu erschließen.

Die Gewinnung von Mandel-Proteinhydrolysat kann nach literaturbekannten Verfahren für die Hydrolyse von Proteinen, z.B. durch saure Hydrolyse, alkalische Hydrolyse oder durch enzymatische Spaltung des in Wasser dispergierten Mandelmehls erfolgen. Bevorzugt geeignete Hydrolysate für die Ausführung der Erfindung werden durch enzymatischen Abbau von Mandelprotein unter Verwendung von Proteasen gewonnen. Mandelprotein ist durch einen hohen Gehalt an sauren und basischen Aminosäuren gekennzeichnet und besteht zu 40 - 50 Gew.-% aus Glutaminsäure, Asparaginsäure und Arginin. Die enzymatische Hydrolyse macht es möglich, unter Verwendung ausgewählter proteolytischer Enzyme selektiv oder bevorzugt Bindungen zwischen ganz bestimmten Aminosäuren hydrolytisch zu spalten und auf diese Weise Oligo- und Poly-Peptide zu erhalten, die bevorzugt ganz bestimmte Endgruppen aufweisen. So werden z.B. in Gegenwart des proteolytischen Enzyms Trypsin bevorzugt der Carboxylgruppen des Lysins und Arginins, durch das Enzym Pepsin bevorzugt die Carboxylgruppen des Leucins, Tyrosins, Phenylalanins und der Glutaminsäure hydrolysiert.

Die enzymatische Hydrolyse kann auch mehrstufig durchgeführt werden, wobei in jeder Stufe die für das jeweils eingesetzte Enzym optimalen Bedingungen in bezug auf pH-Wert und Konzentration eingehalten werden können. Die Verfahren zur enzymatischen Proteinhydrolyse sind literaturbekannt. In Houben-Weyl, "Methoden der organischen Chemie", Band XI/2, (1958), 297 - 298 ist ein Verfahren zur enzymatischen Spaltung von Casein in zwei Stufen unter Verwendung von Pankreatin bei pH = 8 und mit Erepsin (Säugetierdünndarm-Präparat) bei pH = 7,6 beschrieben.

In analoger Weise lassen sich aus Mandelmehl unter Verwendung von proteolytischen Enzymen, z.B. von Trypsin und/oder Pepsin Hydrolysate von definiertem Abbaugrad herstellen.

Bevorzugt geeignet zur Herstellung von erfindungsgemäßen kosmetischen Zubereitungen sind Mandel-Proteinhydrolysate, deren Obligo- und Polypeptide ein mittleres Molekulargewicht von 800 - 4000, bevorzugt von 2000 - 3000 aufweisen. Die enzymatische Hydrolyse des Mandelproteins kann durch pH-Wert-Verschiebung in einen Bereich, bei welchem das verwendete Enzym inaktiviert wird, unterbrochen und das mittlere Molekulargewicht des Hydrolysats auf diese Weise gesteuert werden. Nach Abtrennung der wäßrigen Lösung des hydrolysierten Proteins von ungelösten Rückständen durch Zentrifugation und Filtration wird der Feststoffgehalt zweckmäßigerweise auf einen standardisierten Wert, z.B. von 20 - 25 Gew.-% eingestellt, bei welchem die Lösung noch flüssig ist und mit bekannten wasserlöslichen Konservierungsmitteln konserviert. Eine solche Mandel-Proteinhydrolysat-Lösung enthält ca. 9,5 - 12,5 Gew.-%

Aminosäuren, Oligo- und Polypeptide sowie wasserlösliche Nebenprodukte, die bei der Anwendung in kosmetischen Zubereitungen nicht stören, die gegebenenfalls die kosmetischen Wirkungen des Proteinhydrolysats noch verbessern und daher in dem Proteinhydrolysat verbleiben können. Typisch ist in einem Hydrolysat mit 20 - 25 Gew.-% Gesamt-Feststoff ein Gehalt von ca. 7 Gew.-% Kohlehydraten sowie von Salzen, die einen Aschegehalt von 4 - 5 Gew.-% ausmachen. Der Gehalt an Gesamt-Stickstoff beträgt ca. 1,5 - 2,0 Gew.-%.

Die wasserlöslichen Mandel-Proteinhydrolysate lassen sich den verschiedenen kosmetischen Zubereitungen zur Pflege von Haut und Haaren ohne Probleme zusetzen. Sie sind mit den üblichen Bestandteilen solcher Zubereitungen, insbesondere mit anionischen, ampholytischen, zwitterionischen und nichtionogenen grenzflächenaktiven Stoffen, mit Fett- und Ölkomponenten, wasserlöslichen Polymeren, Duftstoffen, Konservierungsstoffen, Farbstoffen verträglich und lassen sich auch in Emulsionen und Cremes vom Typ Wasser-in-Öl oder Öl-in-Wasser einarbeiten.

Kosmetische Zubereitungen, denen Mandel-Proteinhydrolysate zugesetzt werden können, sind vor allem Hautreinigungsmittel wie z.B. Stückseifen, flüssige Seifen, Duschbadepräparate, Badezusätze (Schaumbade-Präparate) und Hautpflegemittel wie z.B. Hautcremes vom Typ Wasser-in-Öl und vom Typ Öl-in-Wasser, flüssige Emulsionen (Haut-Milch), Gesichtswasser und Haarpflegemittel wie z.B. Haarwaschmittel (Shampoos), Haarnachspülmittel, Haarwellmittel, Haarfärbemittel, Haarbleichmittel und Haarfestigerpräparate.

Besonders vorteilhaft ist die Anwendung der Mandel-Proteinhydrolysate in Zubereitungen zur Reinigung der Haut oder der Haare mit einem Gehalt an oberflächenaktiven Stoffen, insbesondere an schaumstarken anionaktiven Sulfat- oder Sulfonattensiden, d.h. Tensiden, die durch eine bevorzugt lineare Alkylgruppe mit 8 - 18 C-Atomen und durch eine -SO$_3$H-Gruppe oder -OSO$_3$H-Gruppe gekennzeichnet sind. Beispiele für solche Tenside sind z.B. die Natrium-, Kalium-, Ammonium-, Mono-, Di- und Triethanolammoniumsalze von Alkylsulfaten mit 12 - 18 C-Atomen in der Alkylgruppe, von Alkylethersulfaten mit 12 - 16 C-Atomen in der Alkylgruppe und 1 - 10 Glykolethergruppen im Molekül, von α-Olefinsulfonaten und Hydroxyalkansulfonaten, wie sie bei der Sulfonierung von α-Olefinen mit 12 - 18 C-Atomen erhalten werden, von linearen sekundären Alkansulfonaten mit 12 - 18 C-Atomen, von α-Sulfofettsäure (C$_{12}$-C$_{18}$)-methyl-estern, von Sulfobernsteinsäuremonoalkylestern mit 12 - 18 C-Atomen in der Alkylgruppe, von Acylisethionaten und Acyltauriden mit jeweils 8 - 18 C-Atomen in der Acylgruppe. Erfindungsgemäße Haut- und Haarreinigungsmittel enthalten bevorzugt 5 - 25 Gew.-% solcher anionischer Tenside und 0,01 - 5 Gew.-% (Trockensubstanz) des Mandel-Proteinhydrolysats.

Stückseifen auf Basis von Natriumseifen von C$_{12}$-C$_{18}$-Fettsäuren oder flüssige Seifen aus Basis von Kaliumseifen oder Alkanolammoniumseifen von C$_{12}$-C$_{18}$-Fettsäuren oder von Mischungen aus Seifen und synthetischen anionischen Sulfat- oder Sulfonattensiden der o.g. Art können ebenfalls durch Zusatz von 0,01 - 5 Gew.-% (Trockensubstanz) Mandel-Proteinhydrolysat in ihren hautkosmetischen Eigenschaften verbessert werden.

Eine weitere bevorzugte Ausführung der Erfindung sind Haut- und Haarpflegemittel in Form von Emulsionen, entweder vom Typ Wasser-in-Öl oder vom Typ Öl-in-Wasser oder von gemischten Emulsionstypen. Solche Emulsionen enthalten eine Ölphase, in der die kosmetischen Ölkomponenten, Fettkörper, Wachskomponenten, Paraffin und die öllöslichen Emulgatoren enthalten sind, und eine wäßrige Phase, in der die wasserlöslichen kosmetischen Wirkstoffe, z.B. die erfindungsgemäß zu verwendenden Mandel-Proteinhydrolysate, Glycerin, 1.2-Propylenglykol, wasserlösliche Salze (z.B. Puffersalze), wasserlösliche Hydrocolloide, z.B. Carboxymethylcellulose, Hydroxyethylcellulose, Carboxyvinylpolymere (z.B. Carbopol [R]--Typen), Konservierungsmittel, wasserlösliche Emulgatoren und ggf. Farb- und Duftstoffe enthalten sind. Solche emulsionsförmige Hautpflegemittel, z.B. Hautcremes, Hautlotionen, Gesichtsmilch und Haarpflegemittel, z.B. Haarnachspülmittel, Haaravivageemulsionen enthalten bevorzugt 0,01 - 5 Gew.-% Mandel-Proteinhydrolysat zur Verbesserung des Wasserbindevermögens der Haut und der kosmetischen Eigenschaften des Haars.

Die nachfolgenden Beispiele sollen den Erfindungsgegenstand näher erläutern.

Beispiele

In den folgenden Beispielen wurde ein durch enzymatischen Abbau von Mandel-Protein unter Verwendung von Proteasen gewonnenes Hydrolysat mit folgenden technischen Kenndaten eingesetzt:

3

| Feststoffgehalt: | 22 Gew.% |
|---|---|
| Aschegehalt | 4,5 Gew.% |
| Stickstoff (gesamt): | 1,8 Gew.% |
| Oligo- und Polypeptide: | 11 Gew.% |
| Kohlehydrate: | 7 Gew.% |
| mittleres Molekulargewicht der Oligo- und Polypeptide: | ca. 2800 |

Die (nach Totalhydrolyse) ermittelte Zusammensetzung der Aminosäuren war:

| Asparaginsäure | 9,9 Gew.% |
|---|---|
| Threonin | 2,2 Gew.% |
| Serin | 4,1 Gew.% |
| Glutaminsäure | 27,2 Gew.% |
| Prolin | 6,5 Gew.% |
| Glycin | 9,7 Gew.% |
| Alanin | 5,8 Gew.% |
| Cystin | 0,8 Gew.% |
| Valin | 2,9 Gew.% |
| Methionin | 0,4 Gew.% |
| Isoleucin | 2,7 Gew.% |
| Leucin | 5,2 Gew.% |
| Tyrosin | 1,8 Gew.% |
| Phenylalanin | 3,9 Gew.% |
| Histidin | 2,6 Gew.% |
| Lysin | 2,6 Gew.% |
| Arginin | 12,6 Gew.% |

4

Anwendungsbeispiele

1.  Schaumbad

| | |
|---|---|
| Texapon N 25 [1] | 60,0 Gew.-% |
| Cetiol HE [2] | 4,0 Gew.-% |
| Comperlan KD [3] | 1,0 Gew.-% |
| Natriumchlorid | 2,0 Gew.-% |
| Mandelproteinhaydrolysat | |
| (22 %ig) | 2,0 Gew.-% |
| Wasser, Parfüm | ad 100 Gew.-% |

2.  Emulsions-Shampoo mit Ampholyt

| | |
|---|---|
| Texapon N 25 | 20,0 Gew.-% |
| Texapon EVR [5] | 15,0 Gew.-% |
| Dehyton K [6] | 10,0 Gew.-% |
| Mandelproteinhydrolysat | |
| (22 %ig) | 3,0 Gew.-% |
| Wasser, Parfüm | ad 100 Gew.-% |

3.  Schnellhaarkur

| | |
|---|---|
| Cetylalkohol | 2,5 Gew.-% |
| Comperlan KM [4] | 5,0 Gew.-% |
| Dehyquart A [7] | 2,0 Gew.-% |
| Glycerin 86%ig | 5,0 Gew.-% |
| Citronensäure | 1,0 Gew.-% |
| Mandelproteinhydrolysat | |
| (22 %ig) | 2,0 Gew.-% |
| Wasser, Parfüm | ad 100 Gew.-% |

4.  AfterShave-Gel

| | |
|---|---|
| Menthol | 0,2 Gew.-% |

5

| | |
|---|---|
| Ethylalkohol 96%ig | 50,0 Gew.-% |
| Mandelproteinhydrolysat | |
| (22 %ig) | 0,2 Gew.-% |
| Carbopol 940 [16] | 0,8 Gew.-% |
| Triethanolamin | 1,2 Gew.-% |
| Cetiol HE [2] | 5,0 Gew.-% |
| Wasser, Parfüm | ad 100 Gew.-% |

5. Regenerationscreme O/W

| | |
|---|---|
| Cutina KD 16 [8] | 12,0 Gew.-% |
| Emulgin B 1 [10] | 1,0 Gew.-% |
| 2-Octyldodecanol | 10,0 Gew.-% |
| Isopropylmyristat | 6,0 Gew.-% |
| Vitaminöl | 4,0 Gew.-% |
| Henkel Glycerin 86%ig DAB7 | 5,0 Gew.-% |
| Mandelproteinhydrolysat | |
| (22 %ig) | 5,0 Gew.-% |
| Wasser, Parfüm | ad 100 Gew.-% |

6. Hautcreme O/W

| | |
|---|---|
| Cutina MD [9] | 16,0 Gew.-% |
| Emulgin B2 [11] | 3,0 Gew.-% |
| 2-Octyldodecanol | 10,0 Gew.-% |
| 1,2-Propylenglykol | 5,0 Gew.-% |
| Mandelproteinhydrolysat | |
| (22 %ig) | 0,5 Gew.-% |
| Wasser, Parfüm | ad 100 Gew.-% |

7. Hautcreme W/O

| | |
|---|---|
| Dehymuls K [13] | 25,0 Gew.-% |
| Decyloleat | 6,0 Gew.-% |
| Paraffinöl, dickflüssig | 4,0 Gew.-% |
| Vaseline, weiß | 5,0 Gew.-% |

| Magnesiumsulfat-7-hydrat | 0,3 Gew.-% |
| Wasser | 54,7 Gew.-% |
| Glycerin 86 % | 5,0 Gew.-% |
| Mandelproteinhydrolysat | |
| (22 %ig) | 0,3 Gew.-% |
| Wasser, Parfüm | ad 100 Gew.-% |

8. Sonnenschutzcreme O/W

| Cutina MD [9] | 8,0 Gew.-% |
| Cetylstearylalkohol | 2,0 Gew.-% |
| Emulgin B 1 [10] | 1,5 Gew.-% |
| Emulgin B 2 [11] | 1,5 Gew.-% |
| Polywachs 6000 | 3,0 Gew.-% |
| Cetiol LC [14] | 8,0 Gew.-% |
| Isopropylpalmitat | 6,0 Gew.-% |
| Myritol 318 [15] | 6,0 Gew.-% |
| 2-Octyldodecanol | 4,0 Gew.-% |
| Lichtschutzfilter | 4,0 Gew.-% |
| Baysilon-Öl M 300 | |
| (Silikonöl) | 3,0 Gew.-% |
| Glycerin (86 %ig) | 3,0 Gew.-% |
| Mandelproteinhydrolysat | |
| (22 %ig) | 1,0 Gew.-% |
| Wasser, Parfüm | ad 100 Gew.-% |

9. Feuchtigkeitsemulsion O/W

| Cutina MD [9] | 8,0 Gew.-% |
| Emulgin B 1 [10] | 3,0 Gew.-% |
| 2-Octyldodecanol | 10,0 Gew.-% |
| Myritol 318 [15] | 5,0 Gew.-% |
| Paraffinöl, dickflüssig | 4,0 Gew.-% |
| Glycerin 86 % | 5,0 Gew.-% |
| Mandelproteinhydrolysat | |
| (22 %ig) | 1,0 Gew.-% |
| Wasser, Parfüm | ad 100 Gew.-% |

10. Feuchtigkeitsemulsion O/W

| | |
|---|---|
| Cetylstearylalkohol | 3,5 Gew.-% |
| Palmitin-Stearinsäure | 2,0 Gew.-% |
| Emulgin B 3 [12] | 1,5 Gew.-% |
| Paraffinöl | 4,0 Gew.-% |
| 2-Octyldodecanol | 8,0 Gew.-% |
| Mandelproteinhydrolysat (22 %ig) | 3,0 Gew.-% |
| Triethanolamin | 1,8 Gew.-% |
| Carbopol 940 [16] | 0,5 Gew.-% |
| Wasser, Parfüm | ad 100 Gew.-% |

In den Beispielen wurden die folgenden Handelsprodukte verwendet, registrierte Schutzmarken werden als solche anerkannt:

1)  Texapon N25:     Fettalkohol $C_{12}$-$C_{14}$-2EO-sulfat, Na-Salz, 28 %ige Lösung (Henkel KGaA)

2)  Cetiol HE:       Kokosfettsäureester eines Glycerin-Oxethylats (Henkel KGaA)

3)  Comperlan KD:    Kokosfettsäurediethanolamid (Henkel KGaA)

4)  Comperlan KM:    Kokosfettsäuremonoethanolamid (30 %ige Dispersion in Wasser) (Henkel KGaA)

5)  Texapon EVR:     Natriumlaurylethersulfat mit Zusätzen von Fettalkoholsulfat, Fettsäurealkanolamid und Ethy-

EP 0 186 025 B1

lenglykoldistearat (36 %ige
perlglänzende Lösung)
(Henkel KGaA)

6) Dehyton K:    N-Kokosacylamidopropyl-N.N-di-
methylglycin (30 %ige Lösung)
(Henkel KGaA)

7) Dehyquart A:    Cetyltrimethylammoniumchlorid
(25 %ige Lösung)
(Henkel KGaA)

8) Cutina KD 16:    Selbstemulgierendes Gemisch aus
Mono- und Diglyceriden höherer
gesättigter Fettsäuren, vorwiegend Palmitin- und Stearinsäure
mit Kaliumstearat
(Henkel KGaA)

9) Cutina MD:    Gemisch von Mono- und Diglyceriden der Palmitin- und Stearinsäure
(Henkel KGaA)

10) Eumulgin B1:    Anlagerungsprodukt von 12 Mol
Ethylenoxid an Cetylstearylalkohol
(Henkel KGaA)

11) Eumulgin B2:    Anlagerungsprodukt von 20 Mol
Ethylenoxid an Cetylstearylalkohol
(Henkel KGaA)

9

| | | |
|---|---|---|
| 12) | Eumulgin B30: | Anlagerungsprodukt von 30 Mol Ethylenoxid an Cetylstearylal-kohol (Henkel KGaA) |
| 13) | Dehymuls K: | W/O-Salbengrundlage, enthaltend Fettalkohol-Fettsäureester, mineralische Fette und einem Mischester aus Pentaerythrit, Fettalkohol, Fettsäure und Citronensäure (Henkel KGaA) |
| 14) | Cetiol LC: | Capryl-Caprinsäureester von gesättigten $C_{12}$-$C_{18}$-Fettalkoholen (Henkel KGaA) |
| 15) | Myritol 318: | Capryl-Caprinsäure-triglycerid (Henkel KGaA) |
| 16) | Carbopol 940: | Schwach vernetzte Polyacrylsäure (Fa. B.F. Goodrich Company, Chem. Div.) |

**Ansprüche**

1. Verwendung von Mandel-Proteinhydrolysat zur Herstellung kosmetischer Zubereitungen zur Reinigung und Pflege von Haut und Haaren.

2. Kosmetische Zubereitungen zur Reinigung oder Pflege von Haut oder Haaren mit einem Gehalt an oberflächenaktiven Stoffen oder in Form von Emulsionen, dadurch gekennzeichnet, daß ein Mandel-Proteinhydrolysat in einer Menge von 0,01 - 5 Gew.-% Trockensubstanz, bezogen auf die Gesamtzubereitung enthalten ist.

3. Kosmetische Zubereitungen nach Anspruch 2, dadurch gekennzeichnet, daß ein durch enzymatischen Abbau von Mandel-Protein unter Verwendung von Proteasen gewonnenes Hydrolysat mit einem mittleren Molekulargewicht der Oligo- und Polypeptide von 800 - 4000 enthalten ist.

**Claims**

1. The use of almond protein hydrolyzate for the production of cosmetic preparations for the cleansing and care of the skin and hair.

2. Cosmetic preparations for the cleansing or care of the skin and hair containing surfactants or in the form of emulsions, characterized in that they contain an almond protein hydrolyzate in a quantity of 0.01 to 5% by weight dry matter, based on the preparation as a whole.

3.  Cosmetic preparations as claimed in claim 2, characterized in that they contain a hydrolyzate obtained by enzymatic degradation of almond protein using proteases and having an average molecular weight of the oligo- and polypeptides of 800 to 4,000.

**Revendications**

1.  Utilisation d'hydrolysat de protéines d'amandes pour la production de préparations cosmétiques pour le nettoyage et l'entretien de la peau et des cheveux.

2.  Préparations cosmétiques pour le nettoyage et l'entretien de la peau et des cheveux, avec une teneur en substances tensioactives ou sous forme d'émulsions, caractérisées en ce qu'elles contiennent un hydrolysat de protéines d'amandes en quantités de 0,01 à 5% en poids de substances sèches rapportées au total de la préparation.

3.  Préparations cosmétiques selon la revendication 2, caractérisées en ce qu'elles contiennent un hydrolysat obtenu par dégradation enzymatique de protéines d'amandes en utilisant des protéases, avec un poids moléculaire moyen des oligo- et polypeptides compris entre 800 et 4000.